(19) Europäisches Patentamt / European Patent Office / Office européen des brevets

(11) **EP 2 092 062 B1**

(12) **EUROPEAN PATENT SPECIFICATION**

(45) Date of publication and mention of the grant of the patent:
**11.01.2017 Bulletin 2017/02**

(21) Application number: **07819561.7**

(22) Date of filing: **02.11.2007**

(51) Int Cl.:
*C12N 11/14* [(2006.01)]   *G01N 33/58* [(2006.01)]
*C01B 39/02* [(2006.01)]

(86) International application number:
**PCT/EP2007/009538**

(87) International publication number:
**WO 2008/052803 (08.05.2008 Gazette 2008/19)**

(54) **ZEOLITE CRYSTALS WITH BIOLOGICAL MATERIAL**

ZEOLITHKRISTALLE MIT BIOLOGISCHEM MATERIAL

CRISTAUX DE ZÉOLITE AVEC MATÉRIAU BIOLOGIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.11.2006 GB 0621824**

(43) Date of publication of application:
**26.08.2009 Bulletin 2009/35**

(73) Proprietor: **Westfälische Wilhelms-Universität Münster**
**48149 Münster (DE)**

(72) Inventors:
• **DE COLA, Luisa**
**48149 Münster (DE)**
• **POPOVIC, Zoran**
**Cambridge MA 02139 (US)**

(74) Representative: **Hofmann, Harald**
**Sonnenberg Fortmann**
**Patent- und Rechtsanwälte**
**Herzogspitalstrasse 10a**
**80331 München (DE)**

(56) References cited:
**EP-A- 0 594 125      US-B1- 6 503 740**

• LEE Y C ET AL: "ELECTRON MICROSCOPE STUDIES OF THE ABSORPTION OF NATURAL ZEOLITE TO THE PATHOGENIC BACTERIA" ZHONGHUA MINGUO SHOUYI XUEHUI ZAZHI - JOURNAL OF THE CHINESE SOCIETY OF VETERINARY SCIENCE, TAIBEI, TW, vol. 14, no. 1, 1988, pages 65-70, XP008089108 ISSN: 0253-9179
• POPOVIC ZORAN ET AL: "Self-assembling living systems with functional nanomaterials." ANGEWANDTE CHEMIE (INTERNATIONAL ED. IN ENGLISH) 2007, vol. 46, no. 32, 3 July 2007 (2007-07-03), pages 6188-6191, XP002471589 ISSN: 1433-7851
• ALBUQUERQUE RODRIGO Q ET AL: "Luminescence quenching by O2 of a Ru2+ complex attached to zeolite L." CHEMPHYSCHEM : A EUROPEAN JOURNAL OF CHEMICAL PHYSICS AND PHYSICAL CHEMISTRY 12 MAY 2006, vol. 7, no. 5, 12 May 2006 (2006-05-12), pages 1050-1053, XP002471590 ISSN: 1439-4235
• LI HUANRONG ET AL: "Carboxyester functionalised dye-zeolite L host-guest materials" MICROPOROUS MESOPOROUS MATER.; MICROPOROUS AND MESOPOROUS MATERIALS OCT 18 2006, vol. 95, no. 1-3, 18 October 2006 (2006-10-18), pages 112-117, XP002471591
• DEVAUX ET AL: "Solubilisation of dye-loaded zeolite L nanocrystals" MICROPOROUS AND MESOPOROUS MATERIALS, ELSEVIER SCIENCE PUBLISHING, NEW YORK, US, vol. 90, no. 1-3, 29 August 2005 (2005-08-29), pages 69-72, XP005297645 ISSN: 1387-1811

**(Cont. next page)**

- HUBER S ET AL: "Sequential Functionalization of the Channel Entrances of Zeolite L Crystals" ANGEWANDTE CHEMIE. INTERNATIONAL EDITION, WILEY VCH VERLAG, WEINHEIM, DE, vol. 43, 2004, pages 6738-6742, XP002450459 ISSN: 1433-7851 cited in the application

- RUIZ ARANTZAZU ZABALA ET AL: "Organizing supramolecular functional dye-zeolite crystals" ANGEW. CHEM. INT. ED.; ANGEWANDTE CHEMIE - INTERNATIONAL EDITION AUG 4 2006, vol. 45, no. 32, 4 August 2006 (2006-08-04), pages 5282-5287, XP002471592 cited in the application

**Description**

The present invention relates to the combination of biological material and crystalline material. In particular, the invention relates to the combination of zeolite crystals with moieties such as biological cells or viruses.

Assembling molecules in large structures and understanding the type of interactions between molecules and/or a molecule and substrate is increasingly important for the production of molecular devices (see Balzani et al., Topics in Current chemistry, Vol 262:1-27, Springer GmbH, 2005). Recently, nano- and microscale objects such as nanoparticles (see Daniel, Chem Rev 104:293, 2004), micrometer plates (see Clark et al., J Am Chem Soc 123:7677, 2001), and nanorods (see Hurst et al., Angew Chem Int Ed 45:2672, 2006) have been assembled. To date, however, none of the abiotic functional materials such as mesoporous materials or capsules have been covalently or non-covalently attached to living systems in a controllable and selective manner.

It is an object of the present invention to improve the interaction of biological moieties with molecules

Natural zeolites are minerals that are the result of a very low grade metamorphism, typically found in the cavities or vesicles of volcanic rock. They are framework aluminosilicate consisting of interlocking tetrahedrons of $SiO_4$ and $AlO_4$, wherein the corner-sharing $SiO_4$ and $AlO_4$ tetrahedra of the crystalline aluminosilicate give rise to one-dimensional channels arranged in a hexagonal structure. Each aluminium entity in their framework contributes a negative charge that is compensated by an exchange of cations such as sodium, calcium and the like that reside in the large vacant spaces and cages in the structure (see Breck in Zeolite Molecular Sieves, 752, Wiley, New York, 1974; and Baerlocher et al., in Atlas of Zeolite Framework Types, 19, Elsevier, Amsterdam, 5th edition, 2001). The stoichiometry is $(K)_9[Al_9Si_{27}O_{72}] \cdot nH_2O$, where n is 21 in fully hydrated materials and 16 at about 22% relative humidity. Out of 9 potassium cations per unit cell, 3.6 can be exchanged by other monovalent cations, or an equivalent amount of divalent or trivalent cations.

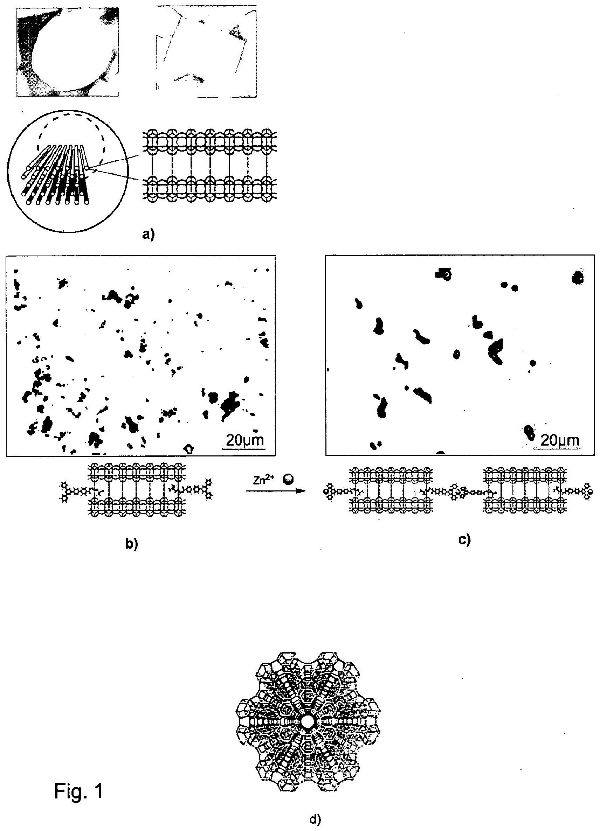

Zeolite L exhibits one-dimensional channels running through the whole crystal, with an opening of 0.71 nm, a largest free diameter of 1.26 nm and a unit cell length of 0.75 nm (see Fig 1d). The centre-to centre distance between two channels is 1.84 nm. As an example a crystal with a diameter of 550 nm consists of about 80,000 parallel channels. Zeolite L channels can be filled with suitable organic guest molecules, although only guests that can pass through the opening are able to enter the channels. Due to the channel entrances, the chemical and physical properties of the base and coat of the cylindrical crystals are different. Exemplary guest molecules include certain dyes possessing desired emission properties (see Calzaferri et al., Angew Chem Int Ed 42:3732, 2003). Zeolite L crystals can also be prepared in different sizes (diameter and length). For example the length of a zeolite L crystal can be from 30 nm to several thousand nanometres (see Ruiz et al., Monatshefte Fur Chemie 136:77, 2005). Pure zeolite L crystals with lengths between 30 and 7000 nm have been synthesized previously (Megelski and Calzaferri, Adv Funct Mater 11:277, 2001). Finally, it is known that the channel entrances within the zeolite L crystal can be chemically modified (see Calzaferri et al., supra), for example by amino derivatives (see Huber et al., Angew Chem Int Ed 43:6738, 2004). In this process, a molecule having a hydrophilic label, a spacer and a head group partially enters a channel of the zeolite L crystal. The label, such as methoxysilane, and the spacer enter the channel allowing the label to attach to the zeolite L crystal. The bulky protection group, e.g. a fluorenylmethylcarbamate group, remains outside the channel entrance due to size restriction imposed by the channel dimensions. Depending on the reactivity of the label, attachment to the crystal can either be reversible or irreversible. The head group is then chemically detached to leave an amino functional group.

Summary of the invention

The invention provides a combination of a monolayer of zeolite L crystals and biological moieties, wherein the biological moiety is selected from the group consisting of biological cells, bacteria and viruses and wherein a first biological moiety is bound to a first side of the monolayer of zeolite crystals by affinity binding agents and a second biological moiety is bound to a second side of the monolayer of zeolite crystals, wherein the second biological moiety is identical or different from the first biological moiety.

The biological cell can be a bacterial, fungal or algal cell. The biological cell can also be a cell from higher organisms such as plant cells, insect cells, and animals cells, especially mammalian cells.

For example, a hybrid construct may be provided comprising at least one zeolite L crystal and at least one cell or virus, wherein the zeolite L crystal has been chemically modified to include means that bind the zeolite L crystal to the biological cell or virus. The cell may be a live cell or the virus may be viable. The cell can be a bacterial, fungal or algal cell. However cells from higher organisms are not excluded and mention may be made of plant, insect and animal cells, especially mammalian cells.

Chemical modification of the zeolite crystal may comprise the binding thereto of one or more affinity binding agent. Affinity binding agents have a binding affinity for the selected target, i.e. the cell or virus. Affinity binding agents can be chosen to have non-specific binding affinities for cells or viruses, or to have a binding affinity for specific structures on the cell or virus, e.g. protein receptors or protein channels provided in the cell membrane. Accordingly, the affinity binding agent can be one partner of any binding partnership known to the skilled person, where the other partner is associated with or is the target on a cell surface or virus. Not wishing to be limited further, but in the interests of clarity, the affinity binding agent may comprise any of the following: an amino group, a charged moiety, hydrogen bonding groups, aromatic moieties, a carbonyl derivative, a thiol group, a cyanate group, a thiocyanate group, a sulfonate or phosphonate group, a hydroxyl group, a halogen, an alkene, an alkyne, proteins, (bio)receptors, sugars, lipid, oligonucleotides, and antibodies and their derivatives, a lectin, an enzyme, a nucleotide, a polynucleotide, a polysaccharide, a receptor agonist, a receptor antagonist, or any combination thereof.

The affinity binding agent may be bound to the crystal via a linker group.

The linker group preferably has a functional group at each end thereof, one capable of binding to the crystal (for example, binding to the silanol groups in the crystal) and the other capable of binding to the affinity binding agent. For example, the linker group can be an organosilane, and preferably conform to the general formula $R_nSiX_{(4-n)}$ (wherein x is the functional group capable of binding to the binding affinity agent (e.g. an alkoxy, halogen, an azide, an alkyne or amino group) and R is a non-hydrolyzable moiety). The linker group may include the affinity binding agent, as is the case with an aminosilane. The linker group may include more than one molecule.

Attachment of the relevant moieties to the crystal via the linker groups may be reversible or irreversible.

The combination according to the invention may further comprises an effector agent. The effector agent can act as an imageable agent or as a biologically active agent. Preferably, the effector agent is located in one or more of the channels of the crystal and is, therefore, preferably sized and dimensioned to be able to fit into the channel. Non-limiting examples include dyes, cations, radionuclides, or biologically active agents such as NO, CO, $H_2$, $NO_2$, antibiotics, amino acids, peptides, hormones (such as steroid hormones) or the like, or any combination thereof. A dye located within a channel can be helpful in assisting visualisation of the construct, and thus provides a means of tracking the attached cell or virus thereof. The dye can be helpful in imaging the cell *in vivo.* A biologically active agent (that is a molecule which can affect a biological system in some way) can enable the zeolite L crystal to be used as a delivery system, such that the effector agent is delivered, for example, to the attached biological cell of the construct. The effector agent could influence differentiation or growth of the cell or could induce cell death or healing. Optionally two or more different effector agents can be present in different channels of the crystal.

Insertion of the effector agent into the channels of the crystal may be achieved by ion exchange, particularly when the effector agent is a cation, by gas phase inclusion, or by crystallisation inclusion.

Alternatively, or in addition, the effector agent can be bound to the outer surface of the zeolite crystal, possibly via a linker group. Effector agents on the surface of the crystal can influence the solubility of the hybrid or influence the transport of the hybrid.

Chemical modification of the crystal may be directed to one or both ends of the zeolite crystal, in close proximity to the opening of the channel, or generally to the outer surface of the crystal. When chemical modification (so as to bind thereto a binding affinity agent) is directed to an end of the crystal, the binding of the crystal to the cell or virus will be orientated such that the channel opening faces the virus or cell. This enables the crystal to administer the effector agent to the cell or virus in a directed manner. In addition, chemical modification to the end of a crystal may provide means for securing the effector agent within the crystal. For example the zeolite crystal can be modified by the binding thereto of one or more stopcock moiety. A stopcock moiety is any moiety that once bound to the crystal at least partially physically inhibits the egress of effector agent from within the channels of the crystal. Preferably, substantially all egress of the effector agent from the channels of the crystal is prevented by the stopcock moiety. Suitable examples of a stopcock moieties are amino groups, carboxylate groups, azide groups, metal ion chelating gourps, a charged moiety, hydrogen bonding groups, aromatic moieties, carbonyl derivatives, a thiol group, cyanate groups, thiocyanate groups, sulfonate or phosponate groups, hydroxyl groups, halogens, an alkenes, an alkynes, proteins, (bio)receptors, sugars, lipids, olygonucleotides, and antibodies and their derivatives, a lectin, an enzyme, a nucleotide, a polynucleotide, a polysaccharide, a receptor agonist, a receptor antagonist, or any combination thereof. The binding affinity agents may be a stopcock moiety. The stopcock moiety may be bound to the crystal via a linker group. The binding of the stopcock moiety to the crystal may be reversible.

A metal ion chelating group may be attached at one end of the zeolite crystal, as a stopcock moiety, such that the entrance of a channel through the zeolite crystal is at least partially blocked. In one embodiment the metal ion chelating group is a terpyridine derivative. The terpyridine derivative can be biphenyl terpyridine (also termed "bitpy"). Zeolite crystals modified by biphenyl terpyridine linked to the crystal via an amino group have been produced and characterised by fluorescence spectroscopy, since the biphenyl terpyridine exhibits an emission at around 350 nm (emission quantum yield, $\Phi$ = 0.45) and with optical microscopy.

Generally it is convenient to introduce the effector agent into the channel of the zeolite crystal prior to modification in the vicinity of the channel entrance.

Chemical modification of the zeolite crystal may be preferentially directed to the ends of the crystal by controlling the ratio of moieties to be bound to the crystal (e.g. stopcock moiety or affinity binding agent) to channel entrances. A ratio of 1:1 or less has been found to favour binding of the moieties to the ends of the crystals. A ratio that includes more moieties than channel entrances results in a more general binding over the surface of the crystal. The average number of channel entrances per given mgs of zeolite L crystals can be calculated as

$$n_e = X_z/l_z \text{ x } 5.21 \text{ x } 10^{-7} \text{ mol}$$

$X_z$ = weight of sample in mg

$L_z$ = average lengths of the zeolite L crystal in nm

The zeolite L crystals naturally carry a negative charge within their channels. It is preferred that modifications such as de-alumination that reduce or eliminate the negative charge of the channel are not applied to the monolayers of crystals of the present invention.

Herein described are also a zeolite crystal, for example a zeolite L crystal, bound to two separate biological cells or viruses. More than one cell or virus may be bound together, to form a complex of cells or viruses, by more than one zeolite crystals. For example, the zeolite crystal may be modified at both ends of the zeolite crystal (in the manner discussed above) so as to attach an affinity binding agent to both ends of the zeolite crystal. Such a construction is capable of binding a cell or virus at each end of the zeolite crystal via the binding affinity agents. When such a hybrid is loaded with an effector agent then both cells or viruses may be co-administered with the same effector agent (when the effector agent is a biologically active agent), or tracked together (when the effector agent is a imageable agent). The zeolite crystal connecting the two cells or viruses may act as a channel through which chemical messengers can pass from one cell or virus to the other. The cells or viruses may be of the same type or may be different from each other. For example, a stem cell may be connected to a differentiated cell via a zeolite crystal.

Such an arrangement can influence the differentiation of the stem cell, by virtue of chemical messengers that are sent from the differentiated cell to the stem cell through the channel of the zeolite crystal.

An array of zeolite crystals comprising at least two zeolite crystals attached together could be attached to a living cell or virus to form the combination. For example, two or more modified zeolite L crystals each having at least one linker group, such as an amino silane, attached thereto may be linked together to form an assembly.

The presence of a chemical reactive group, e.g. an amino group, a charged moiety, hydrogen bonding groups, aromatic moieties, a carbonyl derivative, a thiol group, a cyanate group, a thiocyanate group, a sulfonate or phosponate group, a hydroxyl group, a halogen, an alkene, an alkyne, proteins, (bio)receptors, sugars, lipid, olygonucleotides, and antibodies and their derivatives, a lectin, an enzyme, a nucleotide, a polynucleotide, a polysaccharide, a receptor agonist, a receptor antagonist, or any combination thereof, that is attached to the crystal as part of a linker group and that allows the modified zeolite crystals to assemble into an array (e.g. using condensation reactions, coupling, click chemistry, polymerization, photo/electro reactions, or methatesis reations). Also, non-covalent linkages, such as hydrogen bonds, electrostatic interactions, π-π stackings, can be used to reversible link together the zeolites crystals through a linker group.

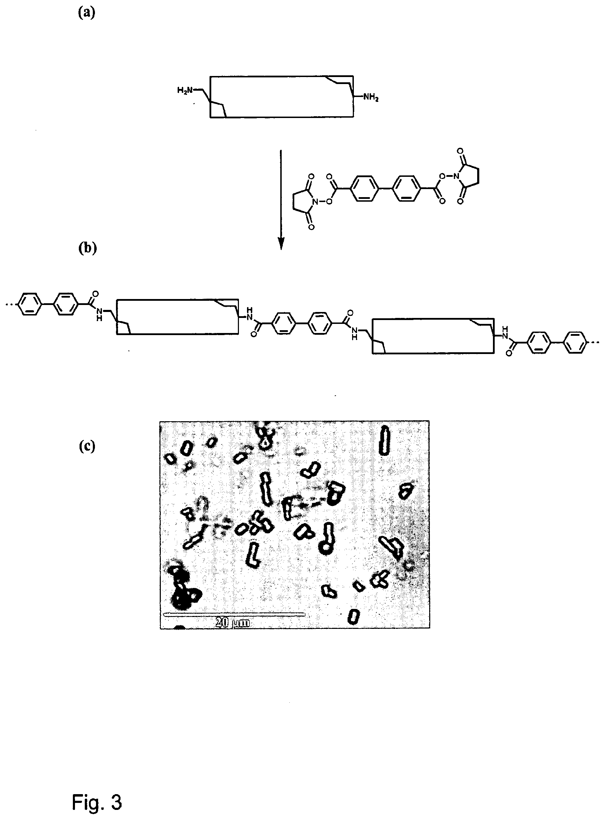

As an example a biphenyl acid derivative (for example a biphenyl boronic acid derivative) is able to link at least two zeolite crystals together after amino functionalisation (e.g. by the binding of an aminosilane to the crystal). Covalently assembled zeolite crystals have been produced and microscopically characterized (Fig 3).

In a further example, two or more modified zeolite crystals each having a linker group that includes at least one metal ion chelating group attached thereto can be bound together after exposure to a cation, such as a divalent metal ion. The presence of an ion allows the modified zeolite crystals to self-assemble into an array, as each ion is able to link two metal ion chelating groups of separate zeolite crystals together. Where the metal ion chelating group is attached in the proximity of a channel entrance in each zeolite crystal, the channel of the crystals will be aligned by chelation of the divalent cation.

Suitable divalent cations include divalent metal ions. Any divalent metal ion can be used, but examples include (but are not limited to) $Zn^{2+}$, $Mn^{2+}$, $Fe^{2+}$, $Ni^{2+}$, $Cu^{2+}$, $Ag^{2+}$, $Co^{2+}$, $Os^{2+}$, $Ru^{2+}$, $Cr^{2+}$, $Pd^{2+}$, and $Cd^{2+}$.

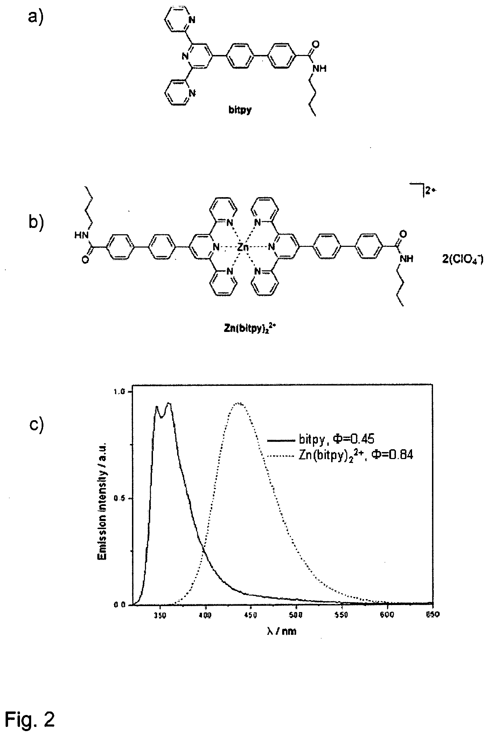

Where the chelating group is biphenyl terpyridine, the use of a metal ion, $M^{2+}$, allows the formation of an octahedral complex, $M(bitpy)_2^{2+}$. Zeolite crystals modified by biphenyl terpyridine linked to the crystal via an amino group and formed into an array using $Zn^{2+}$ have been characterised by fluorescence spectroscopy. Due to the photophysical properties of the $Zn(bitpy)_2^{2+}$ complex so obtained, in particular of its emission wavelength and emission quantum yield ($\lambda$ 450 nm and $\Phi = 0.85$), versus the free biphenyl terpyridine ligand, the reaction can be easily followed using emission spectroscopy or fluorescence microscopy (see Figs 1c and 2).

Other cations with different charge can also be coordinated such as trivalent or monovalent ions.

Alternatively, or in addition, each linker group (attached to separate crystals) to be attached together could comprise an amino silane (such as (3-aminopropyl)triethoxysilane ) and DOTA (1,4,7,10-tetraazacyclodedecane-1,4,7,10-tetraacetic acid). The aminosilane may be bound to the crystal via its ethoxysilane groups, and to the DOTA via its amine groups after activation of DOTA by NHS. Such linkers will bind the crystals together by chelating a common metal ion.

Essentially, therefore, modification of a zeolite crystal according to the present invention by its functionalization with a certain group, e.g. amino, can react with a zeolite functionalized with a complementary group, e.g.carboxy-derivatives. An asymmetric assembly of two crystals can be obtained and this principle of construction can be extended to any other functionalities in which the zeolite crystals contain groups which can react as complementary units.

The metal ion chelating group or linking group may be attached at one end of the zeolite crystal, relative to its longitudinal axis. Thus, lengthwise extension of the zeolite crystal (and of its channels) may be achieved by the binding of two or more of the zeolite crystals together at their ends. The construction of different lengths of the zeolite crystal may be useful in, for example, embodiments where two cells or viruses are bound together via the zeolite crystal; the length of the zeolite crystal will determine the proximity of the cells or viruses to each other.

One or more of the zeolite crystals in the array can contain an effector agent as described above. Optionally, two or more different effector agents can be present in different crystals of the array.

Since each zeolite crystal will have several channels, each of which may be modified by a linker group, multiple attachments between two crystals can be formed at the zeolite-zeolite interface. In the array so-formed the longitudinal axes of the zeolite crystals will be in alignment so that the array is geometrically defined and stable. In one example the array will be a rod-like structure of several tenth of nanometers to hundreds micrometers in length.

Herein described is also an intermediate construct comprising at least one zeolite crystal that has been chemically modified to provide means for binding to a cell or virus. The intermediate construct may include all features of the hybrid construct described herein, in the absence of the cell or virus.

The zeolite crystals could be attached to the cells of higher organism *in vitro* or can be attached or physically adsorbed, to cells which are then introduced back into the body of a patient and either tracked thereafter or deliver the effector agent where required. In this regard particular mention can be made of blood cells (especially erythrocytes, white blood cells such as macrophages, lymphocytes, leucocytes etc) which could be observed directly (e.g. red blood cells). The construct of the present invention could also be used to assess the competence of sperm cells or viruses, attached to the modified zeolite L crystal monolayer.

An arrangement of the combination may be provided. In the arrangement, the zeolite crystals may be substantially arranged in a monolayer. The arrangement may have a first side and a second side. The first side may have a first biological moiety attached thereto. The second side may have a second biological moiety. The second biological moiety may be identical or different from the first biological moiety. The second side may also have an active substrate, such as an electrode or biodegradable material attached thereto.

The zeolite crystal may also be provided as a monolayer of a population of crystals. The zeolite crystals of the monolayer may be modified in the manner discussed above. For example, the zeolite crystals may be modified so as to bind to cells or viruses. Alternatively, the cells can be grown on the monolayer and so are physically associated with the monolayer of zeolite crystals, but are not chemically bound thereto. The crystals of the monolayer can be bound to each other as an array so as to form the monolayer. The monolayer may be associated or bound to a population of cells on both of its surfaces. Where a cell on one side of the monolayer is attached to a cell on the other side of the monolayer by a common crystal, the cells may communicate by the passage of chemical messengers through the common zeolite crystal. In order to achieve such a construction it has been found that one can grow a population of cells on one surface of a monolayer before turning over the monolayer and growing a further population of cells on the other side.

The constructs as described above may be useful in assaying the effects of various effector agents on the biological cell(s) and, where two biological cells are linked together via the zeolite crystal, the effect of the proximity of the cells on each other.

The cells attached to the zeolites crystals can be propagated.

Description of the Figures:

Figure 1 shows a) a Scanning Electron Microscopy (SEM) image of a zeolite L crystal and (within the circle) a schematic representation of the zeolite L crystal indicating that multiple strictly parallel channels are contained within each zeolite L crystal, together with a schematic representation of a longitudinal cross-section of a single channel; b) a schematic cross-section of a single channel within the zeolite L crystal functionalized with biphenyl terpyridine. The optical microscope image shown above the schematic representation shows weak emission, with the crystals appearing as disordered single objects or large disordered aggregates; c) a schematic cross-section of a single channel within two separate zeolite L crystals, each functionalized with biphenyl terpyridine following the addition of $ZnCl_2$. The biphenyl terpyridine groups have chelated a zinc ion, thereby joining the zeolite L crystal such that the channel is aligned in an array. The optical microscope image shown above the schematic representation shows a very intense emission in the blue region and the formation of linear zeolite L crystal arrays; d) shows a schematic representation of a framework of the channels with an opening of 0.71 nm and a hexagonal symmetry.

Figure 2 shows a) a biphenyl terpyridine (bitpy) molecule; b) two bitpy molecules chelating a $Zn^{2+}$ metal ion ($Zn(bitpy)_2^{2+} 2(PF_6)^-$), and c) the emission spectra of bitpy, solid line ($\lambda_{ex}$=295nm) and $Zn(bitpy)_2^{2+}$ $2(ClO_4)^-$, dashed line ($\lambda_{ex}$= 341nm), recorded in air equilibrated dichloromethane.

Figure 3 shows a) an amino-activated zeolite L crystal; b) two zeolite L crystals, one amino group of each amino-activated zeolite L crystal being reacted to the same biphenyl derivative, forming a biphenyl -activated ester induced zeolite L crystal array, and wherein the second amino group of the zeolite L crystal is reacted to an other biphenyl group; and c) an optical microscopy image of the assembled zeolite L crystals, comprising at least two zeolite L crystals per array.

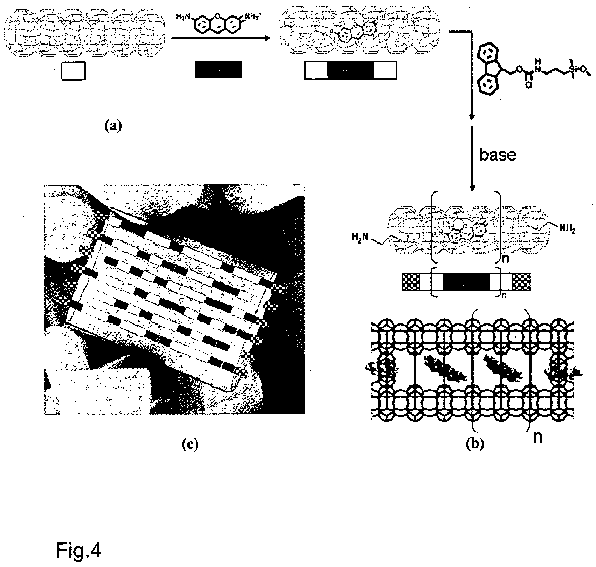

Figure 4 shows a) a schematic representation of a single zeolite L channel (a white box represents a unit cell), the channel having a pyronine molecule (represented by a shaded rectangular) inserted and an amino-functionalized channel having the pyronine molecule inserted (primary amine group: dotted box); and b) a schematic representation of a longitudinal section of an amino-functionalized channel having pyronine molecules inserted, and c) a superimposition of a schematic representation of amino-functionalized channels having pyronine molecules inserted and an SEM image.

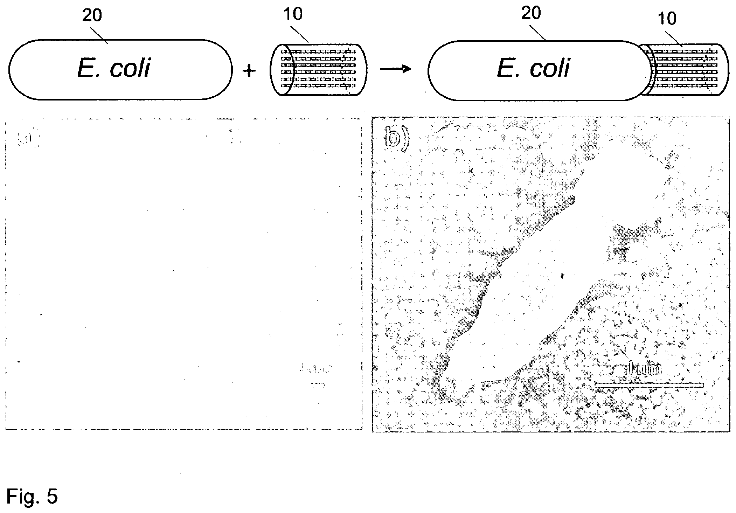

Figure 5 shows a) an optical microscope image taken using white and blue light illumination of a zeolite L crystal construct in PBS buffer solution; and b) a SEM image of the array of Fig 5a after evaporation of the solvent and subsequent coating with silver. Scale bars 1 $\mu$m.

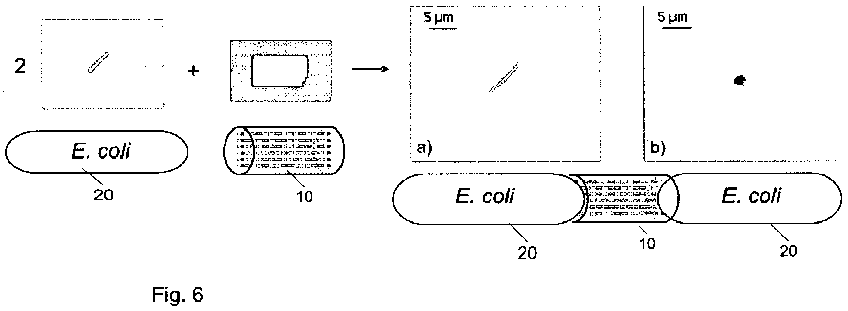

Figure 6 shows a) an optical microscope image taken using white and blue light illumination of a zeolite L crystal construct comprising two bacteria linked by a zeolite L crystal containing pyronine; and b) in the same sample fluorescence of the pyronine filled zeolite (the junction) under blue light.

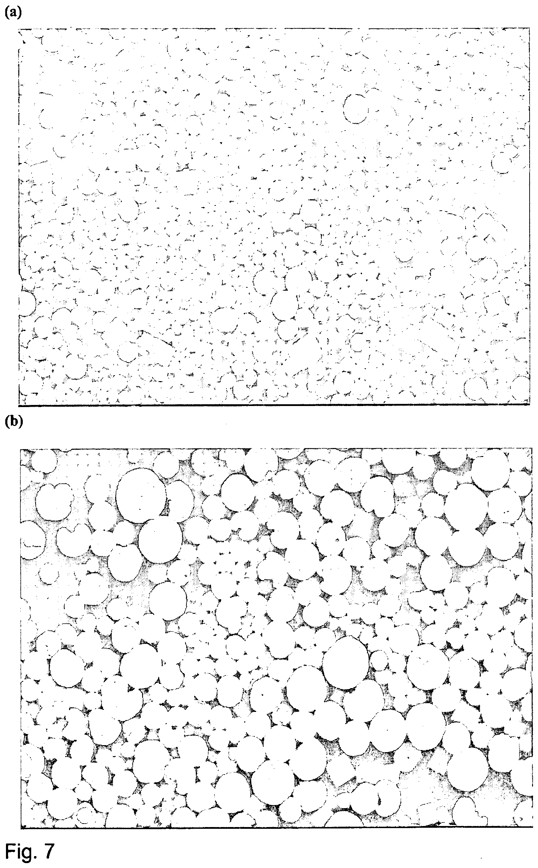

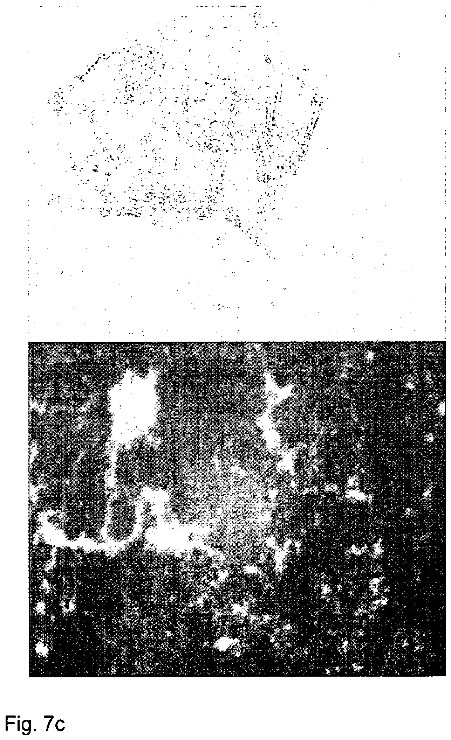

Figure 7 shows a) a SEM image of a monolayer of zeolite L crystals on a Trichlor-(3-cyanpropyl)-Silane (CP-TMS) modified glass plate; b) a magnification of the monolayer as shown in Fig 7a. Figure 7c) shows a Bright field (top) and epifluorescence (bottom) image of Hela cells on disc shaped, thionine intercalated, amino functionalized zeolite crystals taken with a 63X objective.

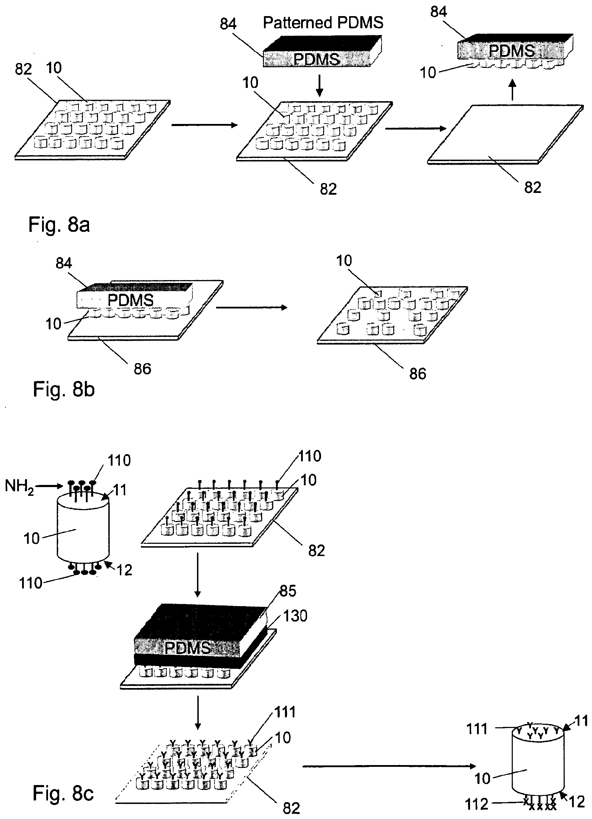

Figures 8a and 8b show the transfer of the zeolite L crystals form one substrate to another.

Figure 8c shows the asymmetric functionalization of the zeolite L crystals.

Microscopy was performed on a Leica inverted microscope and one an Olympus upright microscope. In both cases epifluorescence and bright field microscopy were used. For the nuclei counting experiments 4X objective was used and for the bright filed/epifluorescence 40, 63 and 100X objectives were used.

**Examples**

The cylindrical zeolite L crystals used in this work were of mean length of 2.2 $\mu$m and mean diameter of 1.2 $\mu$m.

Example 1 - Experiments with *E. coli*

A bacterial sample, *E coli* (strain JM109), was freshly prepared from an incubated stock solution (LB medium) and suspended in PBS solution. Concentration was estimated (from optical density) to be in order of $10^9$ cells per ml. A 1 $\mu$m long zeolite crystal was loaded with a green luminescent dye, pyronine, via an ion exchange procedure (see Calzaferri et al., Angew Chem Int Ed 42:3732, 2003). The channel entrances of the zeolite L crystal were then functionalized with amino derivatives as shown in Fig 4 by suspending the zeolite L crystals having a calculated amount of channel entrances in n-hexane (channel entrances: ne = $(X_z/l_z) \times 5.21 \times 10^{-7}$ (ne: number of channel entrances in mols, $X_z$: mass of zeolite in mg, $l_z$: average length of zeolite in nm)). 9-fluorenylmethyl carbamate N-hydroxysuccinimidyl ester was reacted with (3-aminopropyl)methoxydimethylsilane and the reaction product was added to the zeolite L suspension, equalling the amount of channel entrances. The suspension was sonicated (20 minutes) and heated at reflux for 3 hours followed by centrifugation. The modified zeolite L crystals were suspended in dry DMF containing 20% (volume/volume) piperidine, stirred for 1 hour and washed with methanol, resulting in amino terminated zeolite L crystals (see Huber et al., Angew Chem Int Ed 43:6738, 2004). Around 1 mg of the zeolite crystals was suspended in 1 ml of doubly distilled water and sonicated for 15 minutes. Bacteria and the zeolite solutions were mixed in a ratio 1:1 or with the excess of the bacteria (with the respect to the number of bacteria cells and zeolite L crystals; aliquots of 100 $\mu$l) and shaken at 37 °C for 1 hour. After the incubation period an aliquot was taken (10 $\mu$l), diluted 10 times, and the diluted sample deposited (10$\mu$l) on a glass plate. The droplet was covered with a cover microscope glass and sealed. The microscope measurements were performed immediately and the results are shown in Fig 5.

Amino functionalization was selected since it allows the creation of a non-covalent bond between the abiotic material (zeolite) and the living organism. It is believed that electrostatic interactions between the negatively charge surface of the *E. coli* and the protonated amino groups are responsible for the formation of the stable hybrid construct. The primary amino groups are expected to be completely protonated since a pH of about 3.5 is estimated in the channels of the zeolite. Non-functionalized zeolite L crystals do not form stable assemblies with the bacteria. Addition of the pyronine allows easy identification of the zeolite-bacteria hybrid construct by emission of green light after excitation of the sample (see Figs 5a and 5b). The zeolite-bacteria hybrid construct is able to live in physiological conditions and movement is not prevented. The organism is able to swim in the solution despite the attachment to the 1 $\mu$m zeolite L crystal.

Investigation of several samples showed that the zeolite L crystal is attached predominantly to the edge of the bacterium, as shown in Fig 5. This accords with previous observations that micrometer particles attach mainly to the poles of the bacteria (see Jones et al., Appl Env Microb 69:6515, 2003) and is most probably due to a difference in polarity between the poles and the rest of the cell (see Shapiro et al., Science 298:1942, 2002).

## Example 2 - Experiments with *E. coli*

Example 1 was repeated, but the ratio between the bacterial cells and the zeolite L crystals was altered such that the bacteria were present in a large excess over the zeolite L crystal. Constructs in the form of bacteria-zeolite L-bacteria were formed. Fig. 6 shows the results in which the bacteria are clearly visible under white light illumination (Fig. 6a) whilst the fluorescent zeolite L crystal, used as the junction between two of the bacterial cells, is observed when the white light is switched off and only the blue light is used to excite the dye trapped inside the zeolite L crystal (Fig. 6b). The dye encapsulated within the zeolite L crystal exhibited enhanced stability and allowed investigation of the phenomena described here for many hours without any photobleaching being observed.

This example demonstrates that it is possible to use the zeolite L crystal as a scaffold for the attachment of two bacteria. The channels within the zeolite L crystal can be filled with chemicals other than the dye described here.

## Example 3 - Zeolite L crystals assembly with $Zn^{2+}$

Around 10 mg of the bitpy ligand terminated zeolite L crystals (prepared as described in Example 1) were suspended in 1 ml of methanol and warmed to 60°C. A calculated amount (2 eq of zeolite L crystal channel entrances: 1 eq of $ZnCl_2$; calculated as described in Example 1) of the standard solution of $ZnCl_2$ in methanol was added slowly to the stirred suspensions. The reaction mixture was sonicated for 1 minute and stirred for 1.5 days. Centrifugation was performed from methanol yielding the assembled zeolite L crystals that were further dried at 60°C in an oven for 2 hours. The results are shown in Fig 1c.

## Examples 4 - Zeolite L crystal monolayer preparation

A zeolite L crystal 10 monolayer on a glass plate 82 as substrate was prepared according to the Ruiz et al., Angew Chem Int Ed 45:5282-5287, 2006. After a chemical modification of the glass plate with Trichlor-(3-cyanpropyl)-Silane (CP-TMS) by reflux for 3 hours in toluene, the modified glass plates 82 were sonicated for 15 minutes in a toluene suspension of the zeolite L crystals, having a concentration of 1mg/ml, followed by a rinsing step with toluene. The resulting zeolite L crystal monolayer is shown in Fig 7.

Two different types of samples of zeolite L crystals were used:

Type A: Zeolite L crystals having a cylindrical shape of about 1 $\mu$m mean diameter and of about 1 $\mu$m length. Monolayers of cylindrical type A zeolite L crystals 10 were prepared by disposing type A zeolite L crystals without $K^+$ ion exchange on a glass plate 82 according to the procedure described in Ruiz et al., Angew Chem Int Ed 45:5282-5287, 2006.

Type B: Zeolite L crystals having a disc like shape of about 1 $\mu$m mean diameter and about 70 nm length. Monolayers of disc shaped type B zeolite L crystals were prepared by disposing type B zeolite L crystals without $K^+$ ion exchange on the glass plate 82. In some cases, type B zeolite L crystals were intercalated with a thionine dye (emission spectrum in the visible red) and subsequently functionalized at the channel ends with amino groups prior to deposition on the glass substrate. The intercalation was performed as described in Calzaferri et al., J. Phys. Chem. 1992, 96, 3428-3435 and functionalization was performed as described in Huber et al., Angew. Chem. Int. Ed. 2004, 43, 6738-6742; S. Angew. Chem.2004, 116, 6906-6910.

## Example 5 - Zeolite L crystal monolayer preparation

A zeolite L crystal monolayer was prepared as described in example 4 on a quartz plate as the substrate.

## Example 6 - Zeolite L crystal monolayer preparation

A zeolite L crystal monolayer was prepared as described in example 4 on an ITO (Indium-Tin-Oxide) film as substrate.

## Example 7 - Transfer of zeolite L crystals form one substrate to another

A zeolite L crystal monolayer 10 prepared according to any of the examples 4 to 6 was transferred to a micro/nanostructured PDMS (Polydimethylsiloxane) or PMMA (Polymethyl methacrylate) substrate.

A zeolite L crystal monolayer was prepared on the glass substrate 82 as described in Example 4. A PDMS substrate 84 layer is pressed on the zeolite L crystal monolayer 10 on the glass substrate 82 for 1 to 2 minutes and subsequently gently lifted off as shown in Fig. 8a. The pattern is fully transferred to the zeolite L-crystals 10 and a zeolite L crystal pattern is obtained on the PDMS substrate 84. The amount of the zeolite L crystals 10 transferred is almost 100% and the filling of the zeolite L crystal channels does not influence the process.

The zeolite L crystal patterned PDMS substrate 84 is then pressed on a target surface 86, such as ITO (Indium tin oxide), ITO/polymer conductive tapes, gold, silver, silica, or the like to reproduce the zeolite L crystal pattern on the target surface 86 as shown in Fig. 8b. In the case shown, the transfer is not 100%. However, the degree of transfer will depend on the substrate and the substrate functionalization.

An example of the zeolite L monolayer 10 is shown in Fig 8c. The zeolite L crystals in the zeolite L monolayer 10 have a first side 11 and a second side 12 that have each been functionalized with a first group 110 (e.g. amino, carbixylates, azide...) and deposited as the zeolite L monolayer 10 on the glass substrate 82. A PDMS substrate stamp 85 functionalized with a amino active layer 13 complementary to the first group 110 is stamped on the first side 11 of the zeolite L crystals 10. The PDMS substrate stamp 85 has been functionalized with the complementary group. Attaching or stamping the PDMS substrate stamp 85 functionalized with the amino active layer 130 onto the zeolite L crystals leads to a transformation of the first group 110 into a transformed group 111 by a microcontact printing technique as known in the art for surfaces.

By removing the zeolite L crystals in the zeolite monolayer 10 from the substrate 82 the amino groups 112 on the second side 12 of the zeolite monolayer 10 are obtained. The zeolite L crystals in the zeolite monolayer 10 may be detached and removed form the substrate 82 by sonication, possibly followed by centrifugation, washing or other purification methods known in the art.

Example 8 - Experiments with N2a cells

Neuroblastoma cells (N2a) from mouse were used.

After 24h of deposition of the neuroblastoma cells on the substrates, the culture medium was removed, the substrates washed with PBS (Phosphate buffer saline) and fixing mixture (ROTI®-HISTOFIX (FORMALDEHYDLSG. 4,0%) was added. After 15 min, the fixing solution was removed, the substrates were washed with PBS and the nucleus staining dye (HOE-33342 (Bisbenzamide) 2'-(4-Ethoxyphenyl)-5-(4-methyl-1-piperazinyl)-2',5'-bi-1H-benzimidazole·3HCl, from Biomol) was added in water. After 5min the solution was removed, the substrates were washed with water and glued to the microscopy glass plates using a mounting agent. The samples were left in freezer for 2 hours and finally inspected under microscope (bright field and epifluorescence). The stained nuclei were counted and compared between the substrates.

N2a cells were bound to the zeolite L crystals prepared according to any of the method described with respect to Examples 4 to 7.

Example 9 - Experiments with Hela cells

Human Hela (human cervical carcinoma, epithelial like) cells were used. The Hela cells were fixed after 24h in the same way as described in Example 8 with respect to N2a cells except that the Hela cell were not stained. Control Imaging was performed in bright field microscopy.

**Claims**

1. A combination of a monolayer of zeolite L crystals (10) and biological moieties, wherein the biological moiety is selected from the group consisting of biological cells, bacteria and viruses and wherein a first biological moiety is bound to a first side of the monolayer of zeolite crystals (10) by affinity binding agents and a second biological moiety is bound to a second side of the monolayer of zeolite crystals (10), wherein the second biological moiety is identical or different from the first biological moiety.

2. The combination of claim 1, wherein the surface of the zeolite L crystals (10) includes at least one or more affinity binding agents, the at least one or more affinity binding agents preferably being selected from the group consisting of amino groups, azides, peptides, metal complexes, chelating ligands for metal ions, charged moieties, hydrogen bonding groups, aromatic moieties, carbonyl derivatives, thiol groups, cyanate groups, thiocyanate groups, sulfonate or phosponate groups, hydroxyl groups, halogens, alkenes, alkynes, proteins, (bio) receptors, sugars, lipids, oligonucleotides, antibodies and their derivatives, lectins, enzymes, nucleotides, polynucleotides, polysaccharides, receptor agonists, receptor antagonists or any combinations thereof.

3. The combination of any one of the above claims, wherein a biological moiety (20) is bound to the zeolite crystals via a linker group, the linker group being preferably an organosilane, an azide or its derivative, a carbonyl derivative, or any other reactive groups with SiOH or AlOH.

4. The combination of any of the above claims, further comprising an effector agent, the effector agent being preferably a biologically active agent.

5. The combination of claim 4, wherein the effector agent is located in at least one channel of the zeolite crystal, on at least one surface (11,12) or on the coat of the zeolite crystal (10).

6. The combination of any one of the above claims, further comprising a cell growth material in or on at least one channel of the zeolite crystals (10).

7. The combination of any one of the above claims, further comprising at least one stopcock moiety in the at least one channel of the zeolite crystal (10).

8. A method for the manufacture of a monolayer of zeolite crystals (10) comprising:

- depositing a plurality of zeolite crystals (10) on a substrate (82, 86) to form a monolayer of zeolite crystals (10) with a first surface (11) and a second surface (12), the second surface (12) being in contact with the substrates (82, 86);
- binding a first biological moiety (20) to the first surface (11) of the monolayer of zeolite crystals (10);
- attaching a support layer (84) to the first surface (11) of the monolayer;
- removing the substrate (82, 86); and
- binding a second biological moiety (20) to the second surface (12) of the monolayer.

9. The method of claim 14 further comprising filling channels within the zeolite crystals (10) with at least one of the group of ions, small molecules, a growth medium or a combination thereof.

**Patentansprüche**

1. Eine Kombination einer Monoschicht aus Zeolith-L-Kristallen (10) und biologischen Einheiten, wobei die biologische Einheit ausgewählt wird aus der Gruppe bestehend aus biologischen Zellen, Bakterien und Viren und wobei eine erste biologische Einheit über Affinitätsbindemittel an eine erste Seite der Monoschicht aus Zeolithkristallen (10) gebunden ist und eine zweite biologische Einheit an eine zweite Seite der Monoschicht aus Zeolithkristallen (10) gebunden ist, wobei die zweite biologische Einheit mit der ersten biologischen Einheit identisch oder andersartig ist.

2. Die Kombination nach Anspruch 1, wobei die Oberfläche des Zeolith-L-Kristalls (10) mindestens ein oder mehrere Affinitätsbindemittel enthält, und das mindestens eine oder die mehreren Affinitätsbindemittel vorzugsweise ausgewählt werden aus der Gruppe bestehend aus Aminogruppen, Aziden, Peptiden, Metall Komplexen, Chelatliganden für Metallionen, geladenen Einheiten, Wasserstoffbindung bildenden Gruppen, aromatischen Einheiten, Carbonyl Derivaten, Thiolgruppen, Zyanatgruppen, Thiozyanatgruppen, Sulfonat-oder Phosphonatgruppen, Hydroxylgruppen, Halogenen, Alkenen, Alkinen, Proteinen, (Bio-) Rezeptoren, Zuckern, Lipiden, Oligonukleotiden, Antikörpern und deren Derivaten, Lectinen, Enzymen, Nukleotiden, Polynukleotiden, Polysacchariden, Rezeptor Agonisten, Rezeptor Antagonisten oder Kombinationen davon.

3. Die Kombination nach einem der vorherigen Ansprüche, wobei eine biologische Einheit (20) über einen Linker an den Zeolithkristall gebunden ist, und die Linker Gruppe vorzugsweise ein Organosilan, ein Azid oder Azid Derivat, ein Carbonyl Derivat, oder eine andere reaktive Gruppe mit SiOH oder AlOH ist.

4. Die Kombination nach einem der vorherigen Ansprüche, welche zusätzlich ein Effektor-Agens umfasst und das Effektor-Agens vorzugsweise ein biologisch aktives Agens ist.

5. Die Kombination nach Anspruch 4, wobei das Effektor-Agens sich in mindestens einem Kanal des Zeolithkristalls, auf mindestens einer Oberfläche (11, 12), oder auf dem Belag des Zeolithkristalls (10) befindet.

6. Die Kombination nach einem der vorherigen Ansprüche, welche zusätzlich ein Material für Zellwachstum in oder auf mindestens einem Kanal des Zeolithkristalls (10) umfasst.

7. Die Kombination nach einem der vorherigen Ansprüche, welche zusätzlich mindestens eine Stopcock Einheit in dem mindestens einen Kanal des Zeolithkristalls (10) umfasst.

8. Eine Methode zur Herstellung einer Monoschicht aus Zeolithkristallen (10) welche folgende Schritte umfasst:

- Absetzen einer Vielzahl von Zeolithkristallen (10) auf einem Substrat (82, 86) zur Bildung einer Monoschicht aus Zeolithkristallen (10) mit einer ersten Oberfläche (11) und einer zweiten Oberfläche (12), wobei die zweite Oberfläche (12) in Kontakt ist mit den Substraten (82, 86);
- Binden einer ersten biologischen Einheit (20) an die erste Oberfläche (11) der Monoschicht aus Zeolithkristallen (10);
- Anbringen einer Trägerschicht (84) an die erste Oberfläche (11) der Monoschicht
- Entfernen des Substrats (82, 86); und
- Binden einer zweiten biologischen Einheit (20) an die zweite Oberfläche (12) der Monoschicht.

9. Die Methode nach Anspruche 14, welche zusätzlich den Schritt umfasst die Kanäle im Zeolithkristall (10) zu füllen mit mindestens einem Mitglied aus der Gruppe von Ionen, kleinen Molekülen, einem Wachstumsmedium oder einer Kombination der Gruppenmitglieder.

## Revendications

1. Combinaison d'une monocouche de cristaux zéolite L et de fragments biologiques, dans laquelle le fragment biologique est choisi dans le groupe constitué par des cellules biologiques, des bactéries et virus, et dans laquelle un premier fragment biologique est lié à un premier côté de la monocouche de cristaux zéolite (10) par des agents de liaison par affinité et dans laquelle un deuxième fragment biologique est lié à un deuxième côté de la monocouche de cristaux zéolite (10), le deuxième fragment biologique étant identique ou différent du premier fragment biologique.

2. Combinaison selon la revendication 1, dans laquelle la surface des cristaux zéolite L (10) comprend au moins un ou plusieurs agents de liaison, le au moins un ou plusieurs agents de liaison étant choisi de préférence dans le groupe constitué par des groupes amino, azides, peptides, complexes métallique, ligands chélatant sd'ion métallique, fragment de charge, groupes de liants à hydrogène , fragments aromatiques, dérivées carbonyle, groupes thiol, groupes cyanate, groupes thiocyanate, groupes sulfonate or phosponate, groupes hydroxyl,e groupes halogènes, alcènes, alcynes, protéines, (bio) récepteurs, sucres, lipides, oligonucléotides, anticorps and leurs dérivés, lectines, enzymes, nucléotides, poly nucléotides, polysaccharides, récepteur agonistes, récepteur antagonistes ou n'importe quelle combinaison de ceux-ci.

3. Combinaison selon l'une quelconque des revendications précédentes, dans laquelle un fragment biologique (20) est lié aux cristaux zéolite par un groupe lieur, le groupe lieur étant de préférence un organosilane, un azide ou son dérivé, un dérivé carbonyle, ou n'importe quel autre groupe réactif avec SiOH ou AlOH.

4. Combinaison selon l'une quelconque des revendications précédentes, comprenant en outre un agent effecteur, l'agent effecteur étant de préférence un agent biologiquement actif.

5. Combinaison selon la revendication 4, dans laquelle l'agent effecteur est situé dans au moins un canal des cristaux zéolite, sur au moins une surface (11, 12) ou sur un revêtement de cristaux zéolite (10).

6. Combinaison selon l'une quelconque des revendications précédentes, comprenant en outre un matériau de croissance de cellule dans ou sur au moins un canal des cristaux (10).

7. Combinaison selon l'une quelconque des revendications précédentes, comprenant en outre au moins un fragment d'arrêt dans le au moins un canal des cristaux zéolite (10).

8. Procédé de fabrication d'une monocouche de cristal zéolite (10) comprenant :

    - déposer un pluralité de cristaux zéolite (10) sur un substrat (82, 86) pour former une monocouche de cristauxl zéolite (10) avec une première surface (11) et une deuxième surface (12), la deuxième surface (12) étant en contact avec le substrat (82, 86);
    - lier un premier fragment biologique (20) à la première surface (11) de la monocouche de cristal zéolite (10);
    - attacher une couche de support (84) à la première surface (11) de la monocouche;
    - retirer le substrat (82, 86); et
    - lier un deuxième fragment biologique (20) à la deuxième surface (12) de la monocouche.

9. Procédé selon la revendication 14, comprenant en outre le remplissage de canaux à l'intérieur des cristaux zéolite (10) avec au moins un parmi des groupes d'ions, petites molécules, un moyen de croissance ou une combinaison de ceux-ci

a)

b)                                    Zn²⁺  ⊙                              c)

20μm                                                    20μm

Fig. 1

d)

a)

bitpy

b)

Zn(bitpy)₂²⁺

c)

Fig. 2

(a)

(b)

(c)

Fig. 3

(a)

base

(b)

(c)

Fig.4

Fig. 5

EP 2 092 062 B1

Fig. 6

EP 2 092 062 B1

(a)

(b)

Fig. 7

Fig. 7c

Fig. 8a

Fig. 8b

Fig. 8c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BALZANI et al.** Topics in Current chemistry. Springer GmbH, 2005, vol. 262, 1-27
- **DANIEL.** *Chem Rev,* 2004, vol. 104, 293
- **CLARK et al.** *J Am Chem Soc,* 2001, vol. 123, 7677
- **HURST et al.** *Angew Chem Int Ed,* 2006, vol. 45, 2672
- **BRECK.** Zeolite Molecular Sieves. Wiley, New York, 1974, 752
- **BAERLOCHER et al.** Atlas of Zeolite Framework Types. Elsevier, Amsterdam, 2001, 19
- **CALZAFERRI et al.** *Angew Chem Int Ed,* 2003, vol. 42, 3732
- **RUIZ et al.** *Monatshefte Fur Chemie,* 2005, vol. 136, 77
- **MEGELSKI ; CALZAFERRI.** *Adv Funct Mater,* 2001, vol. 11, 277
- **HUBER et al.** *Angew Chem Int Ed,* 2004, vol. 43, 6738
- **JONES et al.** *Appl Env Microb,* 2003, vol. 69, 6515
- **SHAPIRO et al.** *Science,* 2002, vol. 298, 1942
- **RUIZ et al.** *Angew Chem Int Ed,* 2006, vol. 45, 5282-5287
- **CALZAFERRI et al.** *J. Phys. Chem.,* 1992, vol. 96, 3428-3435
- **HUBER et al.** *Angew. Chem. Int. Ed.,* 2004, vol. 43, 6738-6742
- *S. Angew. Chem.,* 2004, vol. 116, 6906-6910